# EUROPEAN PATENT SPECIFICATION

(11) **EP 1 567 500 B1**
(45) Date of publication and mention of the grant of the patent: **25.04.2007**
(21) Application number: 03767959.4
(22) Date of filing: 04.12.2003
(51) Int. Cl.: C07D 215/14

(54) **RESOLUTION OF MEFLOQUINE WITH O,O-DI-P-AROYLTARTARIC ACID**
ENANTIOMERENTRENNUNG VON MEFLOQUIN MIT O,O-DI-P-AROYLWEINSÄURE
RESOLUTION DE MEFLOQUINE AVEC DE L'ACIDE O,O-DI-P-AROYLTARTARIQUE

(30) Priority: 05.12.2002 GB 0228430; 13.12.2002 GB 0229109
(43) Date of publication of application: 31.08.2005
(73) Proprietor: Sosei R&D Ltd., Little Chesterford Saffron Walden Essex CB10 1XL (GB)
(72) Inventor: Baxter, Andrew Douglas, Arakis Ltd., Saffron Walden CB10 1KL (GB); Harris, Michael Christ., Ultrafine, Synergy House, Manchester M15 6SY (GB); Brown, Stuart, Ultrafine, Synergy House, Manchester M15 6SY (GB)
(74) Representative: Perry, Robert Edward
(86) International application number: PCT/GB2003/005286
(87) International publication number: WO 2004/050625

(56) References cited:
- EP-A- 0 808 835
- CARROL ET AL: "Optical isomers of aryl-2-piperidylmethanol antimalarial agents. Preparation, optical purity, and absolute stereochemistry" JOURNAL OF MEDICINAL CHEMISTRY, AMERICAN CHEMICAL SOCIETY. WASHINGTON, US, vol. 17, no. 2, 1974, pages 210-219, XP002093258 ISSN: 0022-2623 cited in the application

## Description

### Field of the Invention

The present invention relates to a process for the manufacture of the single enantiomers of mefloquine.

### Background to the Invention

Mefloquine [*erythro*-α-2'-piperidinyl-2,8-bis(trifluoromethyl)-4-quinolinemethanol] is a chiral drug substance and synthetic analogue of quinine, originally developed to replace existing anti-malarials where resistance had developed. Although mefloquine is marketed as a racemic mixture, the enantiomers of the drug have been shown to demonstrate different biological activities. (+)-Mefloquine has been disclosed (EP-A-0966285) for the treatment of malaria with reduced side-effects. (-)-Mefloquine has been disclosed (EP-A-0975345 and EP-A-01107761) to block purinergic receptors and to have utility in the treatment of movement and neurodegenerative disorders. WO-A-02/019994 discloses that (+)-(11S, 12R)-*erythro*-mefloquine (**1**) 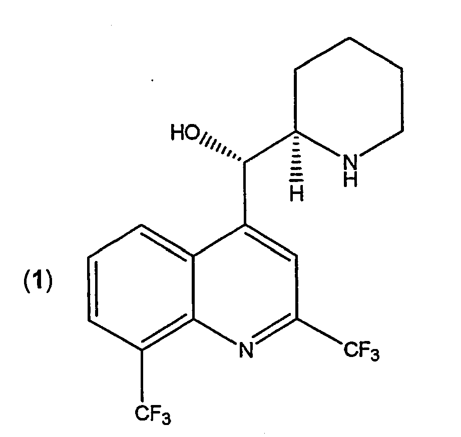 is the preferred enantiomer for the treatment of inflammatory and autoimmune diseases such as rheumatoid arthritis, osteoarthritis, psoriatic arthritis, psoriasis, Crohn's disease, systemic lupus erythematosis (SLE), ulcerative colitis, chronic obstructive pulmonary disease (COPD) and asthma.

An efficient and reliable method for the preparation of the individual enantiomers of mefloquine is desirable. As racemic mefloquine is readily available, a classical resolution process, involving separation of diastereoisomeric salts by selective crystallisation, may be suitable.

Essentially only two routes to the enantiomers of mefloquine are published, but neither is suitable for the preparation of optically pure mefloquine on a commercial scale. Carroll and Blackwell (*Journal of Medicinal Chemistry,* 1974, **17,** 210) resolved the enantiomers of mefloquine by crystallisation with (+)- and (-)-3-bromo-8-camphorsulphonic acid ammonium salts in aqueous methanol. More recently, the asymmetric synthesis of either enantiomer of mefloquine by hydrogenation of a key pyridyl ketone in the presence of a variety of rhodium diphosphine catalysts has been reported (EP-A-0553778 and EP-A-0582632). The intermediate chiral alcohol could be prepared in up to 99% yield and 95% enantiomeric excess. Subsequent hydrogenation over platinum afforded enantiomerically enriched mefloquine in high yield.

### Summary of the Invention

This invention is based on the surprising discovery that racemic mefloquine can be resolved efficiently, using the substantially single enantiomers of *O,O*-di-*p*-toluoyltartaric acid (DTTA) or a related O,O-di-p-aroyltartaric acid as a resolving agent.

### Description of the Invention

The process of this invention may be carried out under conditions that are generally known to those skilled in the art of classical optical resolution methods. Resolutions utilising DTTA are classically carried out in an alcoholic solvent such as methanol or ethanol. However, in the case of mefloquine, methanol, ethanol and butanol proved to be less satisfactory than other solvents. Screening of several solvents indicated that esters, ketones and halogenated solvents, including alkyl alkanoates and haloalkanes, e.g. dichloromethane, methyl isobutyl ketone (MIBK) or isopropyl or ethyl acetate, are capable of providing a single enantiomer of mefloquine in high yield and good enantiomeric excess. Further experiments with ethyl acetate as a solvent indicated that the yield and enantiomeric excess could be further improved by increasing dilution.

In a typical experiment, mefloquine was dissolved in ethyl acetate then treated with a solution of *O,O*-di-*p*-toluoyl-L-tartaric acid monohydrate (1.0 mol equivalent). The resulting solution was allowed to stand until precipitation occurred. Collection of the solid produced the (+)-mefloquine DTTA salt in 40% yield and 98% enantiomeric excess.

Further work demonstrated that better volume efficiencies were achieved when MIBK was the solvent of use. It was then decided to investigate the use of MIBK to identify the best conditions for a reproducible and scalable resolution process. It was found that at a concentration of 8% w/v an optical purity of 91 % was achieved with a 46% recovery. These conditions were selected for further scale-up.

Since both enantiomers of DTTA are readily available in quantity, either can be used to effect the resolution depending on which enantiomer of mefloquine is required. Thus, (-)-mefloquine DTTA salt could be prepared in a similar yield and optical purity utilizing *O,O*-di-*p*-toluoyl-D-tartaric acid monohydrate as the resolving agent.

This resolving agent may also be used to increase the optical purity of enantiomerically-enriched mefloquine. Thus, when both enantiomers of mefloquine are required, the processes described above can be compressed, one enantiomer being recovered by the resolution and the opposite enantiomer being extracted from the mother liquors of the resolution. In practice, when (+)-mefloquine DTTA salt is recovered as described above, the mother liquors remaining are processed to isolate mefloquine free base enriched in the (-)-isomer, which is then purified by treatment with *O, O*-di-*p*-toluoyl-D-tartaric acid monohydrate and crystallization of the resultant salt.

In a preferred embodiment of the invention, resolution of *erythro-*mefloquine may be conducted using a sub-stoichiometric quantity of, say, D or L-ditoluoyltartaric acid, thereby preferentially crystallising (+) or (-)-*erythro-*mefloquine enantiomers. This procedure is preferably conducted in the presence of an additional chiral or achiral acid.

Other beneficial aspects of the process of the present invention can be summarized as follows:
1. The DTTA resolving agent can be easily recovered in a state of high purity, such that it can be re-used in one or more subsequent resolution processes.
2. If desired, less than 1.0 molar equivalent of DTTA may be used in the process.
3. Efficient resolution can be achieved when the input racemic mefloquine is contaminated with the isomeric threo-mefloquine.

A substantially single enantiomer that is used in or produced by the process of the invention may be in at least 80% ee, preferably at least 90% ee, more preferably at least 95% ee, and most preferably at least 98% ee.

The present invention is illustrated by the following Examples.

### Example 1 (+)-(11S, 12R)-Erythro-mefloquine, O,O-(-)-ditoluoyl-L-tartrate salt

To a solution of (-)-*erythro*-mefloquine (10.0 g mmol, 26.6 mmol) in ethylacetate (440 mL) was added a solution of *O,O*-(-)-ditoluoyl-L-tartaric acid (10.2 g, 26.4 mmol, 1.0 equiv.) in ethyl acetate (80 mL). This corresponds to a 4% solution w/v. The resulting solution was stoppered and stirred at room temperature for 3 hours. The white crystalline solid formed was filtered off and washed with ethyl acetate (2 x 200 mL), and dried under vacuum to furnish 8.69 g, of product. The solid was then suspended in ethyl acetate (220 mL) and heated under reflux for 1 hour. On cooling the solid was filtered, washed with ethyl acetate (100 mL) and dried under vacuum to furnish the product as a colourless solid 6.88 g, yield 34 %, 98.3 % ee.

### Example 2 (+) - (11S, 12R)-Erythro-mefloquine

The isolated product of Example 1 (6.66 g, 8.74 mmol) was suspended in methanol (22 mL). A solution of water (92 mL) and 22 % sodium hydroxide (6.7 mL) was charged over 1 hour until a final pH 14 was reached. The suspension was stirred at room temperature for 2.5 hours and the suspension filtered, washed with water (2 x 50 mL) and dried under vacuum to furnish the (+)-(11S, 12R)-*erythro*-mefloquine as a colourless solid, 2.94 g, yield 89 %, 98.8 % ee.

### Example 3 (+)-(11S, 12R)-Erythro-mefloquine, hydrochloride salt

The isolated product of Example 2 (2.78 g, 7.34 mmol) was dissolved in diisopropyl ether (110 mL) and stirred at room temperature. A solution of 2 N hydrochloric acid in diethyl ether (4 mL) was added dropwise with stirring and the resulting suspension stirred at room temperature for 1 hour. The suspension was filtered and the solid washed with diisopropyl ether (2 x 100 mL) and dried to furnish (+)-*erythro* mefloquine hydrochloride as a colourless solid, 2.86 g, yield 94 %, >99 % ee.

### Example 4 Free base formation

(±)-Mefloquine hydrochloride (60.0 g, 144.6 mmol) was suspended in 1 M aqueous sodium hydroxide solution (500 ml) in a separating funnel. Ethyl acetate was added (300ml), after shaking, the layers were separated. The aqueous layer was extracted a further two times with ethyl acetate (2 x 300 ml) and the combined organics dried (MgSO₄) then reduced to dryness to give (±)-*erythro* mefloquine as a white solid. 53g, yield 97 %.

### Example 5 General procedure for resolution - small scale

(±)-*Erythro* mefloquine (500 mg, 1.32 mmol) was dissolved in of MIBK and a solution of *O,O*-di-*p*-toluoyl-D-tartaric acid monohydrate (511 mg, 1.32 mmol, 1.0 equiv.) dissolved in 1 ml in MIBKwas added. (-)-Erythro mefloquine *O, O*-di-*p*-toluoyl-D-tartaric acid salt (5mg) was added as seed crystals.

The solution was allowed to stir for 3 hours then left overnight to stand at room temperature, after which any solid was filtered under reduced pressure and washed with the relevant solvent (2 x 5 ml) to yield (-)-*erythro* mefloquine *O, O*-di-*p*-toluoyl-D-tartaric acid salt. The combined supernatant and washings were reduced to dryness to give a white powder.

### Example 6 20g scale

(*±*)-*Erythro* mefloquine (20 g, 52.91 mmol) was dissolved in 204.4 ml of MIBK and a solution of the *O, O*-di-*p*-toluoyl-D-tartaric acid monohydrate (20.44 g, 52.91 mmol, 1.0 equiv.) dissolved in 200 ml in MIBK was added. (*-*)-*erythro* mefloquine *O,O*-di-*p*-toluoyl-D-tartaric acid salt (5mg) was added as seeds crystals.

The solution was allowed to stir for 3 hours then left overnight to stand at room temperature, after which any solid was filtered under reduced pressure and washed with the relevant solvent (3 x 50 ml) to yield the (-)-erythro mefloquine *O, O*-di-*p*-toluoyl-D-tartaric acid salt. The combined supernatant and washings were reduced to dryness to give a white powder.

## Claims

1. A process for increasing the optical purity of a mixture of enantiomers of mefloquine, using a substantially single enantiomer of *O, O*-di-*p*-aroyltartaric acid as a resolving agent, and which is conducted in a solvent selected from esters, ketones and halogenated solvents.

2. A process according to claim 1, for preparing a substantially single enantiomer of mefloquine from racemic mefloquine.

3. A process according to claim 1 or claim 2, wherein the resolving agent is *O,O-*di-*p*-toluoyl-L-tartaric acid.

4. A process according to any preceding claim, wherein the mixture of enantiomers of mefloquine is contaminated with *threo*-mefloquine.

5. A process according to any preceding claim, wherein the solvent is dichloromethane, methyl isobutyl ketone, isopropyl acetate or ethyl acetate.

6. A process according to any preceding claim, wherein the resolving agent is present in a sub-stoichiometric quantity, whereby an enantiomer of *erythro*-mefloquine is preferentially obtained.

7. A process according to claim 6, which is conducted in the presence of an additional chiral or achiral acid.

8. A process according to any preceding claim, which further comprises conversion of the salt obtained by the resolution to the free base form of mefloquine or a pharmaceutically acceptable salt thereof.

## Patentansprüche

1. Verfahren zum Erhöhen der optischen Reinheit einer Mischung von Enantiomeren von Mefloquin unter Verwendung eines im Wesentlichen einzigen Enantiomers von O,O-Di-p-aroylweinsäure als Auftrennungsmittel, das in einem Lösungsmittel durchgeführt wird, das aus Estern, Ketonen und halogenierten Lösungsmitteln ausgewählt ist.

2. Verfahren nach Anspruch 1 zur Herstellung eines im Wesentlichen einzigen Enantiomers von Mefloquin aus racemischem Mefloquin.

3. Verfahren nach Anspruch 1 oder Anspruch 2, in dem das Auftrennungsmittel O,O-Di-p-toluoyl-L-weinsäure ist.

4. Verfahren nach irgendeinem vorangehenden Anspruch, in dem die Mischung von Enantiomeren von Mefloquin mit threo-Mefloquin kontaminiert ist.

5. Verfahren nach irgendeinem vorangehenden Anspruch, in dem das Lösungsmittel Dichlormethan, Methylisobutylketon, Isopropylacetat oder Ethylacetat ist.

6. Verfahren nach irgendeinem vorangehenden Anspruch, in dem das Auftrennungsmittel in einer unterstöchiometrischen Menge vorliegt, wodurch ein Enantiomer von erythro-Mefloquin bevorzugt erhalten wird.

7. Verfahren nach Anspruch 6, das in Anwesenheit einer zusätzlichen chiralen oder achiralen Säure durchgeführt wird.

8. Verfahren nach irgendeinem vorangehenden Anspruch, das weiter die Umwandlung des durch die Auftrennung erhaltenen Salzes in die freie Basenform von Mefloquin oder ein pharmazeutisch annehmbares Salz derselben umfasst.

## Revendications

1. Procédé pour l'augmentation de la pureté optique d'un mélange d'énantiomères de mefloquine avec utilisation d'un énantiomère substantiellement unique d'acide O,O-di-p-aroyltartrique comme agent de résolution, et qui est mis en oeuvre dans un solvant choisi parmi les esters, les cétones et les solvants halogénés.

2. Procédé selon la revendication 1 pour la préparation d'un énantiomère substantiellement unique de mefloquine à partir de mefloquine racémique.

3. Procédé selon la revendication 1 ou la revendication 2, dans lequel l'agent de résolution est l'acide O,O-di-p-toluoyl-L-tartrique.

4. Procédé selon l'une quelconque des revendications précédentes, dans lequel le mélange d'énantiomères de mefloquine est contaminé avec de la *thréo*-mefloquine.

5. Procédé selon l'une quelconque des revendications précédentes, dans lequel le solvant est le dichlorométhane, la méthylisobulylcétone, l'acétate d'isopropyle ou l'acétate d'éthyle.

6. Procédé selon l'une quelconque des revendications précédentes, dans lequel l'agent de résolution est présent en une quantité sous-stoechiométrique, dans lequel un énantiomère d'*érythro-*mefloquine est préférentiellement obtenu.

7. Procédé selon la revendication 6, qui est mis en oeuvre en présence d'un acide chiral ou achiral supplémentaire.

8. Procédé selon l'une quelconque des revendications précédentes, qui comprend en outre la conversion du sel obtenu par la résolution en la forme base libre de la mefloquine ou d'un sel pharmaceutiquement acceptable de cette dernière.
